# EUROPEAN PATENT SPECIFICATION

(11) **EP 2 306 920 B1**
(45) Date of publication and mention of the grant of the patent: **07.11.2018**
(21) Application number: 09790343.9
(22) Date of filing: 13.07.2009
(51) Int. Cl.: A61B 90/11, A61B 90/00, A61B 34/30, A61B 17/34

(54) **APPARATUS FOR INTRODUCING A MEDICAL DEVICE INTO THE BODY OF A PATIENT**
VORRICHTUNG ZUR EINFÜHRUNG EINES MEDIZINISCHEN INSTRUMENTS IN DEN KÖRPER EINES PATIENTEN
APPAREIL POUR INTRODUIRE UN DISPOSITIF MÉDICAL DANS LE CORPS D'UN PATIENT

(30) Priority: 11.07.2008 US 80193 P
(43) Date of publication of application: 13.04.2011
(73) Proprietor: Houston Medical Robotics, LLC, Houston TX 77058 (US)
(72) Inventor: HERLIHY, J. Patrick, Houston, TX 77005 (US); COHN, William E., Bellaire, TX 77401 (US); RENNICKS, K. Wayne, Pearland, TX 77581 (US)
(74) Representative: Zimmermann & Partner Patentanwälte mbB
(86) International application number: PCT/US2009/050415
(87) International publication number: WO 2010/006335

(56) References cited:
- WO-A1-96/20024
- WO-A1-99/01174
- WO-A1-99/49793
- WO-A2-2006/120619
- WO-A2-2009/092059
- US-A1- 2004 267 121
- US-A1- 2005 033 177
- US-A1- 2007 185 485

## Description

### BACKGROUND OF THE INVENTION

### Field of the Invention

Embodiments of the present invention generally relate to an image guided hand held robotic medical device. Particularly, embodiments of the present invention relate to methods and apparatus for introducing a medical device into a body. More particularly, embodiments of the present invention relate to methods and apparatus for cannulating a bodily cavity. More particularly still, embodiments of the present invention generally relate to use of a robotic device to percutaneously place a central venous catheter into the central veins, or to place an introducer needle into a central vein, thereby facilitating placement of a central venous catheter.

### Description of the Related Art

Central venous catheters (also referred to herein as "CVC") are used for a variety of purposes. In one application, CVCs are placed to allow rapid infusion of fluids, especially blood products in critically ill patients. CVCs also allow small aliquots of blood to be removed for various tests, such as complete blood count. In addition, the external hubs of the catheters can be connected to pressure monitoring equipment to allow measurement of central venous pressures, important data in critically ill patients.

A common approach to placing a percutaneous CVC follows a procedure developed by Swedish radiologist Sven Seldinger in the 1950s. The procedure generally involves a series of manually performed steps that have remained largely unchanged to date. First, a hollow introducer needle is manually inserted through the skin and placed in the vein. Second, a guide wire is manually inserted through the hollow of the needle into the lumen of the vein. The guide wire is inserted until a portion of the guide wire extends past the end of the needle. In this position, the distal end of the wire is in the central vein and the proximal end is outside the patient's body. The introducer needle, which at this point has the guide wire running through its length, is then removed from the patient by pulling the needle out and over the wire. During removal of the needle, the distal end of the guide wire is undisturbed inside the lumen of vein. Third, the hollow CVC is placed over the proximal end of the guide wire, and the CVC advanced along the wire, through the skin, the subcutaneous tissues, and into the vein. At its final position, the catheter will have one end in the vein and the other end outside of the body. The guide wire can now be retrieved by pulling the guide wire through the catheter and out of the body, without disturbing the position of the catheter. The catheter can now be used to access to the central venous circulation.

However, insertion of the catheter using this procedure still faces many challenges. For example, the practitioner has to rely on various surface landmarks to estimate the location of the vein for insertion of the introducer needle. In this respect, the insertion process may require multiple attempts in order to achieve the proper position. In addition, during each attempt, the practitioner must simultaneously create negative pressure by withdrawing the plunger on the syringe until the practitioner can visually confirm the source of the blood, *i.e.,* venous blood. Moreover, the practitioner must avoid accidentally puncturing adjacent structures such as lung, artery, lymphatic tissues, and others, depending on the location of the target structure.

More recently, ultrasound has been used to assist in the manual placement of a CVC in a vein. Even though ultrasound can locate the venous lumen and provide a visual target, the CVC still requires manual placement using the Seldinger technique. Thus, even with ultrasound guidance, failure to properly place the CVC and complications resulting therefrom are still a common occurrence.

For example, document WO 2006/120619 describes a highly automated puncture system for inserting a cannula or a needle into a blood vessel of a person or an animal. The puncture system has an acquisition module that allows for determining at least a location of a blood vessel underneath of the surface of a skin and is further enabled to determine an optimal puncture location that is well suitable for inserting a cannula into the blood vessel. Further, the puncture system has an actuator for moving and aligning the cannula to a determined position. The system is further adapted to autonomously insert the cannula into the blood vessel for multiple purposes, such as blood withdrawal, venous medication and infusions.

There is a need, therefore, for apparatus and methods to visualize the lumen of the intended blood vessel and directly place a catheter in the lumen of blood vessel or other bodily cavity. There is also a need for a robotic device to cannulate one or more structures in a human. There is a further need for an image guided hand held robotic device to introduce a medical device into the anatomy of a human.

### SUMMARY OF THE INVENTION

In light of the above, the image guided device according to independent claim 1 is provided. Further advantages, features, aspects and details are evident from the dependent claims, the description and the drawings.

Embodiments of the present invention provide an image guided robotic device to perform a diagnostic or therapeutic medical procedure. In one embodiment, the robotic device includes an imaging machine, an actuator, and a controller for controlling the actuator. The robotic device may be configured to introduce a tubular shaped device such as a needle, a catheter, or a cannula into an anatomical structure of a human body. The device and its components may be sized for use as a portable device and/or operable using one hand of the operator. The anatomical structure may be any portion of the body of diagnostic or therapeutic interest.

In one embodiment, the robotic device may be used to position a tubular device into a target vessel. The robotic device is provided with ultrasound capability to detect and image the target vessel. The controller is adapted to determine the distance to the target vessel using the ultrasound image and information. The controller may activate the actuator to introduce the tubular device such as a needle, catheter, or cannula into the target vessel.

In another embodiment, the robotic device may be used to introduce a medical device having an elongated portion for insertion into the anatomy. The elongated portion may have a solid interior, hollow interior such as a bore, or combinations thereof. In one embodiment, the elongated portion may have a cross-section shape such as elliptical, circular, polygonal, or any suitable cross-section for performing the intended diagnostic or therapeutic procedure.

In another embodiment, a robotic device for introducing a medical device into an anatomical structure includes an imaging device for acquiring information of a location of the anatomical structure; an actuator for moving and introducing the medical device; and a controller for processing information of the location of the anatomical structure and controlling the actuator to introduce the medical device into the anatomical structure. The robotic device may be portable. The robotic device includes a cartridge containing the medical device, wherein the cartridge is releasably attached to the actuator. In one embodiment, the medical device is a hollow or solid device having a polygonal, round, or elliptical cross-section.

In one embodiment, the medical device may be retrieved after a procedure is performed. In another embodiment, the medical device may remain in the body as needed. In yet another embodiment, a tubular medical device may be used to deliver another medical device or a chemical compound for diagnostic or therapeutic purposes. In yet another embodiment, the medical device may be used to withdraw a solid or fluid bodily material.

In another embodiment, the robotic device may be used to cannulate a vessel using the Seldinger technique. The device may be configured to position a needle, then a flexible guide wire, and finally, the catheter into the target vessel.

In another embodiment, an image guided device for introducing a medical device into a body structure includes an imaging device for acquiring information of a location of the body structure; an actuator for deploying the medical device; and a controller for processing information of the location of the body structure and controlling the actuator to place the medical device into the body structure. In another embodiment, the imaging device includes a monitor.

In another embodiment, an image guided device for introducing a tubular into a bodily cavity includes a console having a monitor; a handle coupled to the console; and an actuator pivotally coupled to the console, wherein the actuator includes a motor to introduce the tubular in the bodily cavity. In another embodiment, the monitor may be provided with crosshairs to facilitate targeting of the bodily cavity.

A method of cannulating a bodily cavity includes determining a path of a needle; simultaneously injecting the needle and a catheter into the bodily cavity, wherein the catheter is co-axially disposed around the needle; inserting a wire through the needle and into the bodily cavity; advancing the catheter relative to needle and at least partially over the wire; and retrieving the needle and the wire.

In another embodiment, an image guided robotic device for placing a medical device in a bodily cavity includes an imaging device for acquiring information of the bodily cavity; a housing coupled to the imaging device; a cartridge releasably installed on the housing, wherein the cartridge contains the medical device; and a drive mechanism for deploying the medical device.

A method of cannulating a bodily cavity includes viewing an image of the bodily cavity; determining a path of a needle; injecting the needle into the bodily cavity according to the image; inserting a wire through the needle and into the bodily cavity; advancing a catheter relative to the needle and at least partially over the wire; and retrieving the needle and the wire. The method may further include disposing the catheter co-axially with the needle prior to injecting the needle. The needle may be injected using a robotic device. The robotic device may be equipped with a monitor to display the image.

### BRIEF DESCRIPTION OF THE DRAWINGS

So that the manner in which the above recited features of the present invention can be understood in detail, a more particular description of the invention, briefly summarized above, may be had by reference to embodiments, some of which are illustrated in the appended drawings. It is to be noted, however, that the appended drawings illustrate only typical embodiments of this invention and are therefore not to be considered limiting of its scope, for the invention may admit to other equally effective embodiments.
Figure 1 is a perspective view of an exemplary image guided robotic medical device. As shown, the robotic device has been configured to place a catheter into a vein.
Figure 2 is a top view of the robotic device of Figure 1.
Figure 3 is a top view of an exemplary cartridge containing a catheter suitable for use with the robotic device of Figure 1.
Figures 4A-7A show an exemplary operational sequence of positioning a catheter in a vessel using the device of Figure 1.
Figures 4B-7B show a partial top view of the operational sequence corresponding to Figures 4A-7A.
Figure 8 shows the catheter placed in a vessel using the operational sequence of Figures 4A-7A.
Figure 9 is a top view of another exemplary robotic device configured for catheter placement.
Figure 10 is another view of the robotic device of Figure 9.
Figure 11 is a bottom view of the robotic device of Figure 9.
Figures 12A-B are side view and top view, respectively, of an exemplary catheter containing cartridge suitable for use with the robotic device of Figure 9.
Figures 13A-C show an embodiment of a locking mechanism for engaging the catheter cartridge.
Figures 14A-C show an embodiment of a catheter gripper suitable for use with the device of Figure 9.
Figures 15-19 show an exemplary operational sequence of positioning a catheter in a vessel using the device of Figure 9.

### DETAILED DESCRIPTION

Embodiments of the present invention provide an image guided robotic device for introducing a medical device into the anatomy of a human such as a hollow fluid or gas filled structure. In one embodiment, the robotic device may be adapted to place a needle or catheter into the bodily structure. Particularly, the robotic device may be configured to initially place a hollow needle, then a flexible guide wire, and finally, the catheter. The robotic device may be provided with ultrasound capability to detect the hollow fluid structure. The robotic device and its components may be sized for use as a portable device and/or operable using one hand of the operator.

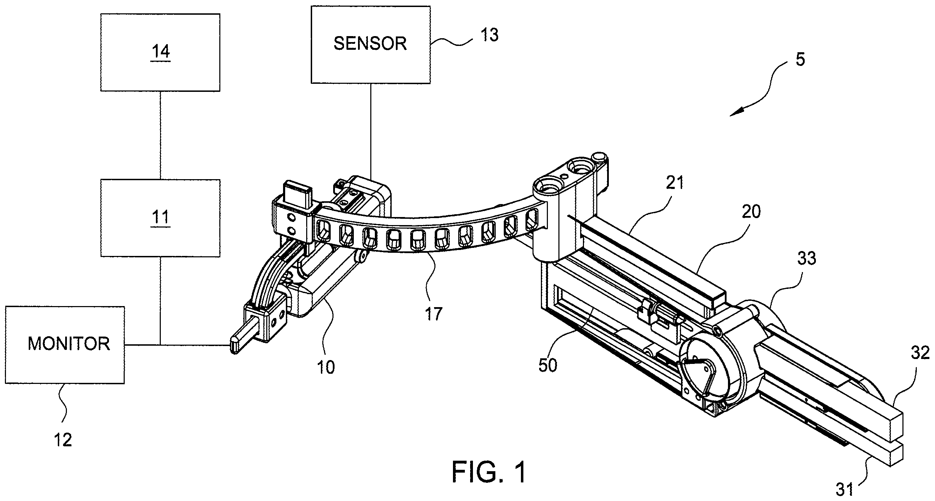

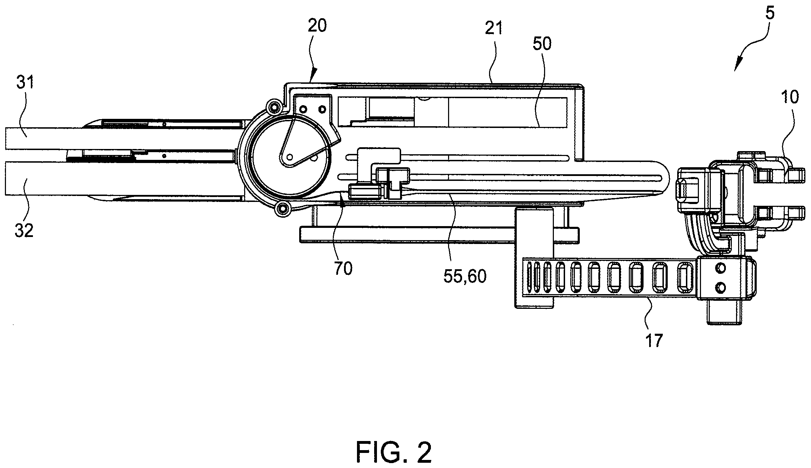

Figures 1-2 show another embodiment of an image guided robotic device 5 for placing a catheter or needle into a lumen of a blood vessel. The device 5 has a console 10 and an actuator 20 for deploying the needle and/or the catheter. Figure 2 shows a top view of the device 5. The console 10 includes an imaging machine 11, a monitor 12, and a controller 14 for controlling the actuator 20. A handle may be attached to the actuator housing 21 for handling of the device 5. One or more control buttons for operating the device 5 may be positioned within the reach of the operator's hand holding the handle. In one embodiment, the device 5 may have two control buttons; one for starting the entire operation and one for emergency stop. In another embodiment, the control buttons are positioned for manipulation by the thumb of the hand gripping the handle. In yet another embodiment, the control buttons may be located on the actuator housing or adjacent the monitor for activation by the user's other hand. In this respect, the user may control the x and y motion of the device 5 with one hand while supporting the procedure with the other hand. The use of both hands would provide consistent pressure to ensure high fidelity with regard to imaging during operation of the device 5. In another embodiment still, the handle may serve as a receptacle for a battery to provide power to the device 5. The battery may be any suitable energy storage unit, including disposable or rechargeable batteries.

The imaging machine 11 of the device 5 may be an ultrasound machine or any suitable imaging machine for detecting a target such as the lumen of the vessel.

Exemplary imaging machines are disclosed in U.S. Pat. No. 6,068,599, issued on May 30, 2000, to Saito et al., and U.S. Pat. No. 6,132,379, issued on Oct. 17, 2000, to Patacsil et al.

The imaging function may be used to delineate the geometry and position of the target vessel. In one embodiment, the ultrasound machine 11 has Doppler signaling for locating and targeting of the central vein below the skin. The ultrasound machine 11 may send and receive signals to and from the central vein through the sensor 13 located at the lower portion of the console 10. In one embodiment, the sensor 13 may comprise an ultrasound transducer that transmits and receives ultrasonic waves. The ultrasonic transducer may be any suitable type known to a person of ordinary skill in the art, such as a piezoelectric transducer formed of one or more piezoelectric crystalline material arranged in a two or three dimensional array. The ultrasound machine 11 may analyze the ultrasound data and display the result on the monitor 12. In one embodiment, the monitor 12 may be sized for mounting on the console 10 such that the monitor 12 is portable with the device 5. For example, the size of the monitor 12, as measured diagonally, is from 5,08 cm to 25,4 cm (2 in. to 10 in.); preferably, from 7,62 cm to 17,78 cm (from 3 in. to 7 in.); and more preferably, from 8,89 cm to 15,24 cm (3.5 in. to 6 in.).

In another embodiment, the device 5 may have attachments for connection to an independent monitor. In this respect, the device 5 may utilize monitors positioned at different locations. The monitors 12 may display a target zone such as a crosshair viewable by the operator. Viewing the monitor 12, the operator may move the device 5 until the desired position (e.g., lumen) for the needle is aligned with the crosshair on the monitor 12. Use of the crosshair may allow for precise targeting in the lumen of the vessel, such as the center of the lumen. Alternatively, the target zone in the vein may be determined by the programmable logic in the controller. It is contemplated that other suitable imaging machines such as computerized tomography (CT) and magnetic resonance imaging (MRI) may be used to acquire an image or location information of the target.

The controller 14 is adapted to process the ultrasound data and control the actuator 20 to deploy the needle to the central vein. In one embodiment, the controller 14 may include programmable logic to process information from the imaging machine
11 and determine the distance to the lumen. The controller may also include programmable logic to cause the actuator 20 to deploy the needle, guide wire, and/or the catheter into the lumen. In one embodiment, the position of the actuator 20 on the device 5 is fixed such that the device 5 is preset to introduce the medical device to the location identified by the crosshair. In another embodiment, the controller may control activation of the actuator and adjust the azimuth angle of the actuator 20 for penetration into the skin and direct the needle to the location identified by the crosshair. A suitable controller may be a microprocessor.

The robotic device 5 is equipped with the necessary electronics for the controller 14 to carry out its functions. In one embodiment, the controller 14 may include internal or external memory, which may be any suitable type. For example, the memory may be a battery-backed volatile memory or a non-volatile memory, such as a one-time programmable memory or a flash memory. Further, the memory may be any combination of suitable external and internal memories. Additionally, controller 14 may include a program memory and a data memory. The program memory may store a motor control sequence and the data memory may store a data log. The motor control sequence may be stored in any data format suitable for execution by the controller 14. For example, the motor control sequence may be stored as executable program instructions. A power system may be provided to operate the controller 14. The power system may include a power controller, power supply, and a power transducer, as is known to a person of ordinary skill in the art. Power may be supplied through a battery or a battery charger. Other suitable electronics may be provided as is know to a person of ordinary skill in the art.

The actuator 20 may be pivotally connected to the console 10. The actuator 20 may pivot relative to the console 10 to adjust the azimuth of the needle for insertion. As shown, the console 10 is connected to the actuator 20 using a support arm 17. The support arm 17 may be arcuate in shape. One or both of the console 10 and actuator 20 may pivot relative to the support arm 17. The console 10 may also move along the support arm 17 to adjust a distance between the console 10 and the actuator 20.

The actuator 20 contains the drive mechanisms for deploying the needle, guide wire, and/or the catheter. Referring again to Figures 1 and 2, the actuator 20 includes a housing 21 for receiving the catheter cartridge 50 and for supporting the motors to carry out the operation. The device 5 may include a first injector motor 31 for advancing or retracing the needle and a second injector motor 32 for advancing or retracing the catheter relative to the needle. An exemplary injector motor is a linear motor. The linear motors 31, 32 may be provided with force feedback and position sensing capabilities such that the location of the needle or catheter may be determined at anytime. The device 5 also includes a rotary motor 33 for advancing or retracing the guide wire. Each of the motors may be operated in both the forward and the reverse directions. Operation of the motors may be performed by the controller/microprocessor 14 in response to information obtained from the imaging machine 11 and/or the force feedback and position information from the motors 31, 32, 33.

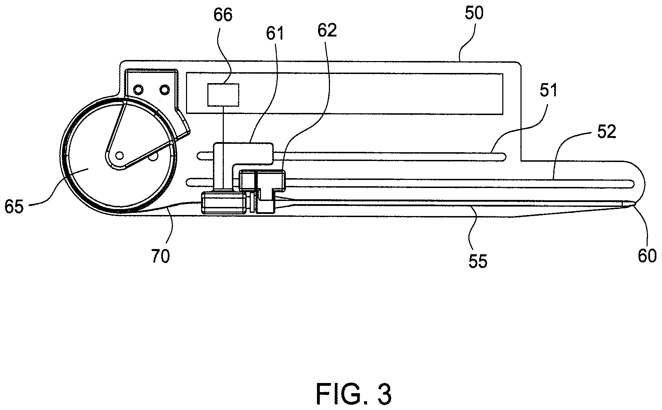

Figure 3 is a top view of an exemplary catheter cartridge 50. The cartridge 50 may be positioned in the device housing 21 and connect to the motors for operation. As shown, the catheter cartridge 50 includes the catheter 55, the needle 60, and the spool 65 containing the guide wire 70. The catheter 55 is coaxially disposed around the shaft of the needle 60. The upper portion of the needle 60 is attached to a first holder 61. The first holder 61 is movable in a slotted track 51 of the catheter cartridge 50. The bottom of the first holder 61 has an adapter that connects to the first linear motor 31 when the cartridge 50 is positioned in the device housing 21. In this respect, the first motor 31 is actuatable to move the first holder 61 along the track 51. In one embodiment, the adapter may be a fin-like structure engageable with a mating aperture in the motor 31.

The upper portion of the catheter 55 is attached to a second holder 62. The second holder 62 is positioned below first holder 61 on the needle 60 and is moveable in a second slotted track 52 of the catheter cartridge 50. The bottom of the second holder 62 has an adapter that connects to the second linear motor 32 when the cartridge 50 is positioned in the device housing 21. The catheter 55 may be made of an elastomer or any suitable catheter material known to a person of ordinary skill. In one embodiment, the catheter 55 is releasably attached to the second holder 62 to facilitate release of the catheter 55 from the cartridge 50 after placement in the target vessel. It must be noted that the position of the two slotted tracks relative to the needle 60 may be varied so long as the second holder 62 holding the catheter 55 is positioned below the first holder 61. It is contemplated that any suitable number of motors may be used to accomplish the procedure. For example, one linear motor may be used to position both the needle 60 and catheter 55. After the needle 60 is positioned in the vessel, the linear motor may release the first holder 61 and grip the second holder 62 and thereafter advance the catheter 55 relative to the needle 60.

The guide wire 70 may be wound around the spool 65 on the cartridge 50. The spool 65 is rotatable by the rotary motor 33 to advance or retract the guide wire 70. In one embodiment, the tip of the guide wire 70 is pre-positioned in the bore of the shaft of the needle 60 before deployment. An exemplary guide wire 70 is a flexible "J" tipped guide wire.

The cartridge 50 may optionally include a vacuum mechanism 66 for aspirating a bodily fluid. The collected bodily fluid may be analyzed to confirm the fluid is venous blood. In one embodiment, the vacuum mechanism 66 is a syringe in fluid communication with the needle 60. A syringe actuator may be provided to pull back on the syringe to draw in a fluid sample from the needle 60. In another embodiment, one or more sensors may be provided in the chamber of the syringe, the needle, or the vacuum mechanism 66 to analyze the fluid. Alternatively, the fluid sample may be visually confirmed.

An exemplary procedure for placing the catheter 55 using the image guided robotic device 5 will now be described. It should be noted that prior to performing the procedure, the desired area of operation on the patient should be prepped in the usual sterile fashion. A sterile field will be established using drapes and a sterile ultrasound media will be applied to the general area to be determined by the operator. A sterile ultrasound cap made of thin plastic may be wrapped around the device. The cartridge 50 prevents exposure to the sharp implements of the device.

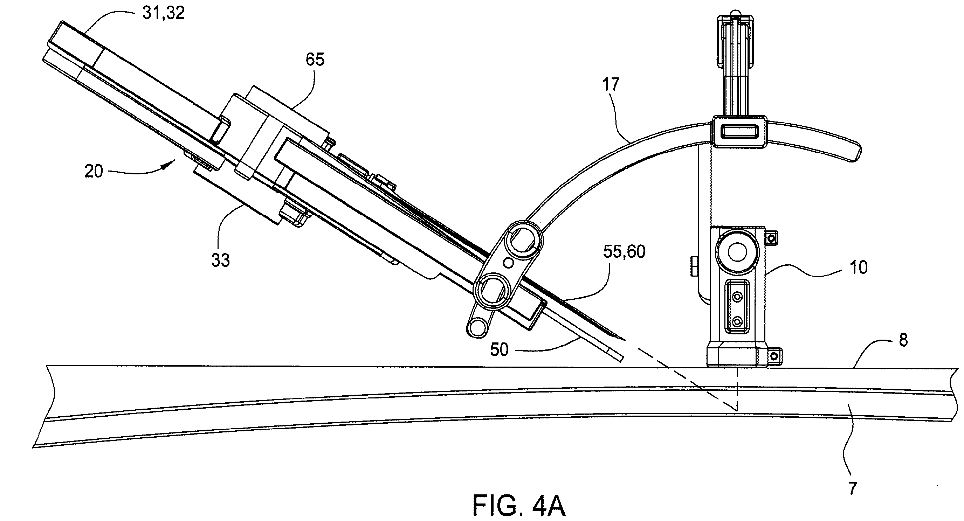

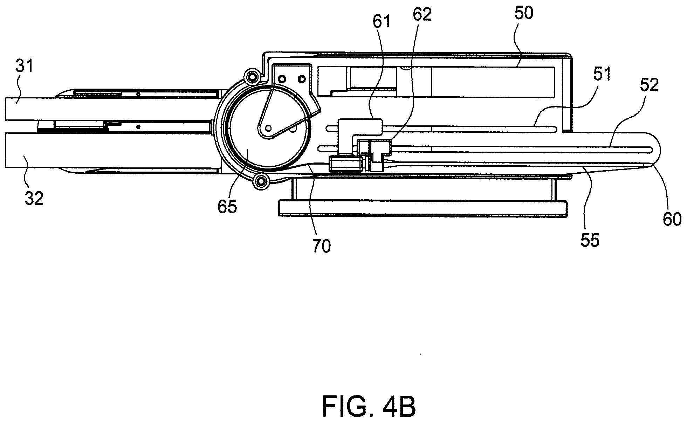

In operation, a cartridge 150 is releasably positioned on the actuator 20 of the device 5. The adapters on the bottom of the holders 61, 62 and the spool 65 engage the motors 31, 32, 33 of the actuator 20. Using ultrasound imaging, the device 5 identifies the target lumen, which, in this example, is the central vein 7. The device 5 is moved by the operator until a precise location in the target lumen is aligned with the crosshair on the monitor 12. Additionally, the imaging function allows the operator to select a path for the needle 60 to avoid accidentally puncturing any adjacent structures. The console 10 or the actuator 20 may be pivoted to facilitate identification of the target lumen. The target sets the floor or maximum penetration depth. As shown in Figure 4A, the sensor 13 is directed toward the lumen and the needle 60 is aimed at the lumen and ready to penetrate the skin 8. Figure 4B is a partial top view of the device 5 without the console 10. Figure 4B shows the cartridge 50 disposed on the actuator 20 and ready for deployment. Prior to insertion, the surgically prepared skin surface 8 will be exposed to an anesthetic. The anesthetic may be delivered manually or by an optional component of the device 5.

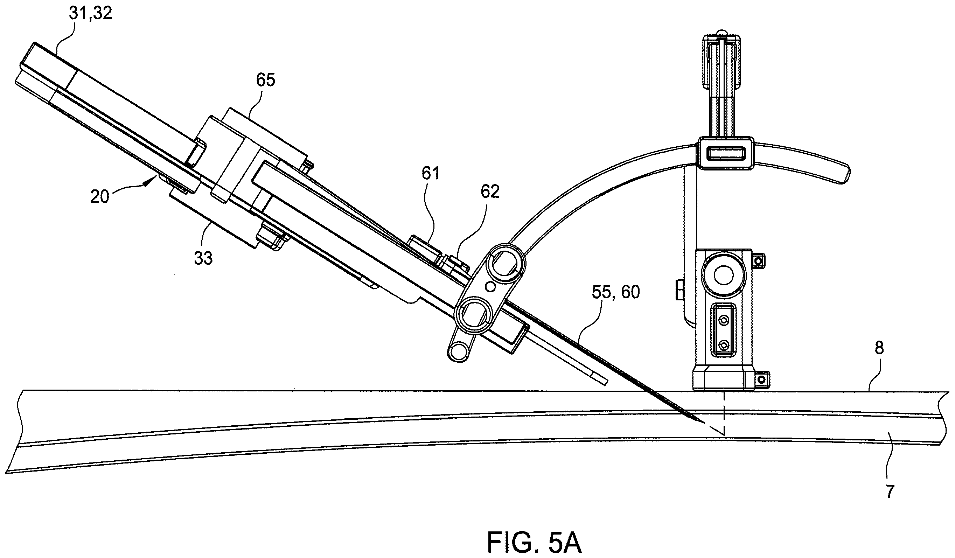

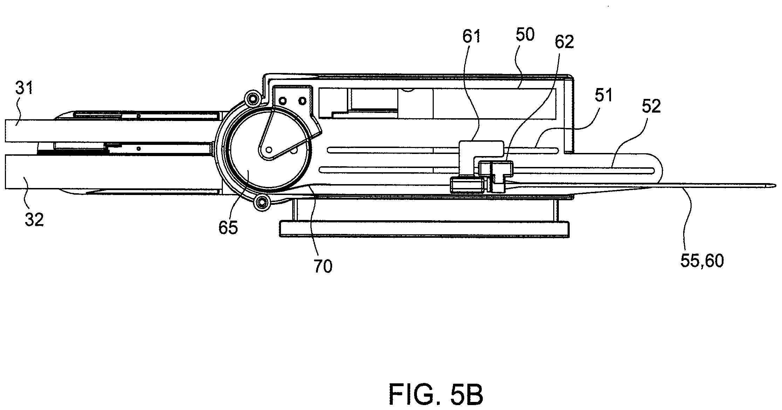

Figure 5A shows introduction of the needle 60 and the catheter 55 into the vessel 7. The actuator 20 has pivoted to the proper angle for insertion of the needle 60 and the co-axially disposed catheter 55. From the position in Figure 4A, the operator instructs the controller 14 to activate the first linear motor 31 to advance the needle 60 and the catheter 55. In this respect, the first linear motor 31 simultaneously advances the first holder 61 and the second holder 62 down the first track 51 and the second track 52. The insertion is stopped by the controller 14 when the needle 60 has penetrated the vessel 7 to the proper depth identified by the crosshair. The controller 14 may stop advancing the needle 60 based on a predetermined distance traveled or the force feedback received from the motor 61, or combination thereof. In this manner, the catheter 55 and the needle 60 are inserted into the vessel 7 simultaneously. Because the guide wire 70 was partially positioned in the needle 60, the guide wire 70 is spooled out along with the insertion of the needle 60. Figure 5A shows the tip of the needle 60 in the lumen of the vessel 7. Figure 5B is a partial top view of the device 5 showing the positions of the needle 60 and the catheter 55 on the cartridge 50 after insertion of the needle 60.

After insertion, the controller 14 may optionally activate the vacuum mechanism 66 to aspirate a sample of bodily fluid into the needle 60. Aspiration may also remove air from the bore of the needle 60. The fluid sample may be analyzed by the sensor to confirm the fluid is venous fluid, e.g., venous blood. In another embodiment, fluid confirmation may be visually performed by the operator. In yet another embodiment, the device 5 may be equipped with a pressure sensor to detect the puncture of a blood vessel by the needle.

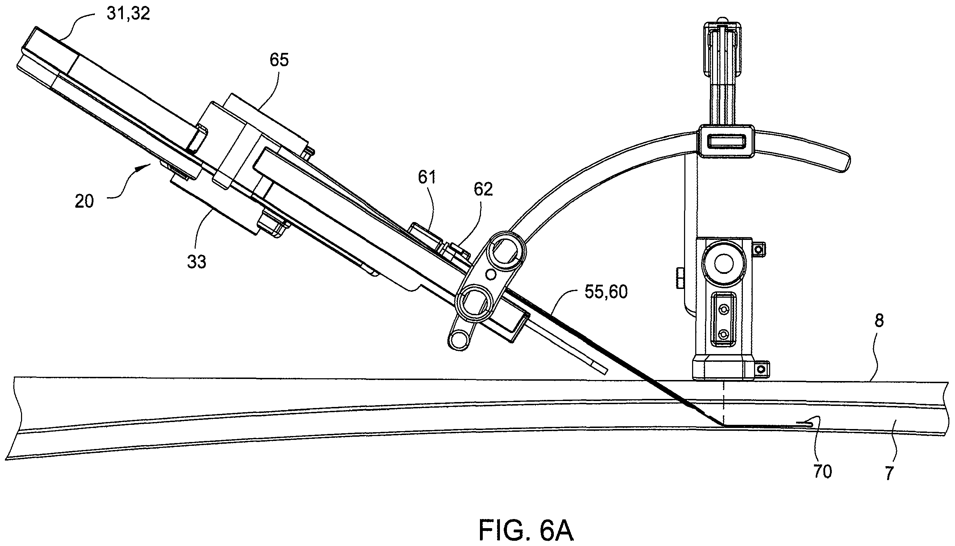

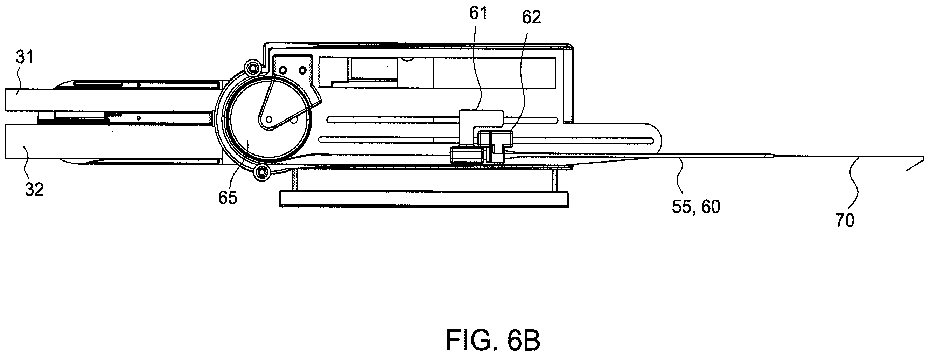

Thereafter, the rotary motor 33 is activated to advance the flexible "J" tipped guide wire 70 through the needle 60 and into the vein 7, as shown in Figure 6A. The guide wire 70 is extended sufficiently as to ensure that the catheter 55 does not subsequently extend past the flexible "J" tip of the guide wire 70. Figure 6B is a partial top view of the device 5 showing the positions of the needle 60, the catheter 55, and the guide wire 70 on the cartridge 50 after insertion of the guide wire 70.

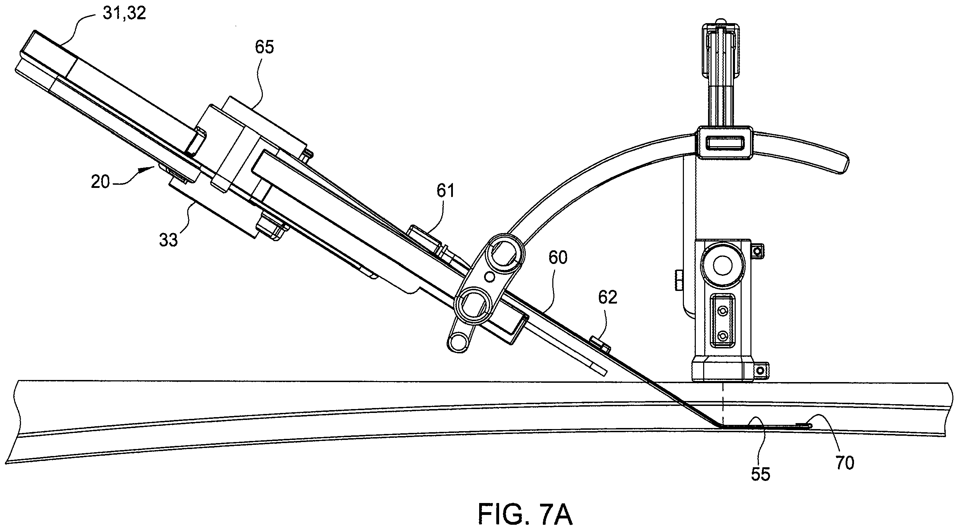

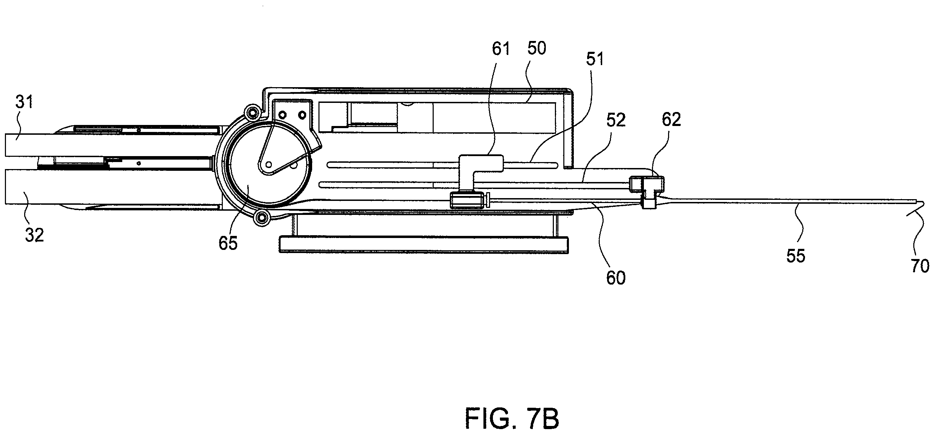

Referring to Figure 7A, after the guide wire 70 has extended past the needle tip, the second linear motor 32 is activated to advance the second holder 62 and the catheter 55. The catheter 55 is at least partially advanced past the needle 60 and over the guide wire 70. Advancement of the catheter 55 by the second linear motor 32 is relative to the needle 60. Figure 7A shows the catheter 55 advanced along the needle 60 and over the guide wire 70. In this respect, the second holder 62 has moved away from the first holder 61. From the partial top view of Figure 7B, the second holder 62 is shown advanced down the second track 52 while the first holder 61 remains in position. Thereafter, the catheter 55 is released from the second holder 62.

After placement of the catheter 55, the needle 60 and the guide wire 70 are retrieved. The first linear motor 31 is activated to retract the needle 60 back within the perimeter of the cartridge 50. Also, the rotary motor 33 may be reversed to wind the guide wire 70 back onto the spool 65. In one embodiment, during needle retraction, negative pressure aspiration is repeated to aspirate from within the catheter 55 as the needle 60 sheds the catheter 55. In this respect, air in the catheter 55 may be removed.

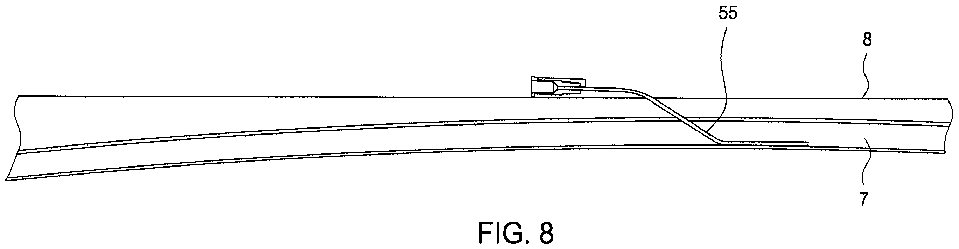

The cartridge 50 may now be ejected from the device housing 21. The cartridge 150 may be ejected into an appropriate disposal unit. The robotic device 5 may be used again with a new cartridge. Figure 8 shows one end of the catheter 55 placed in the vein 7 and the other end on the skin surface 8.

In one embodiment, the placed catheter 55 may be flushed with fluid, such as 0.9% normal saline, and the operator may apply a pinch clamp to create a positive pressure environment within the catheter to ensure that blood or fluid does not migrate from the vein into the catheter.

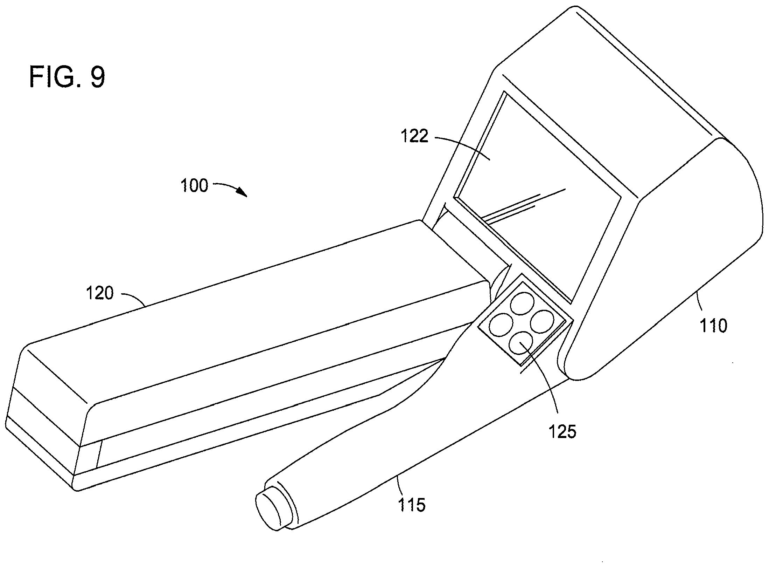

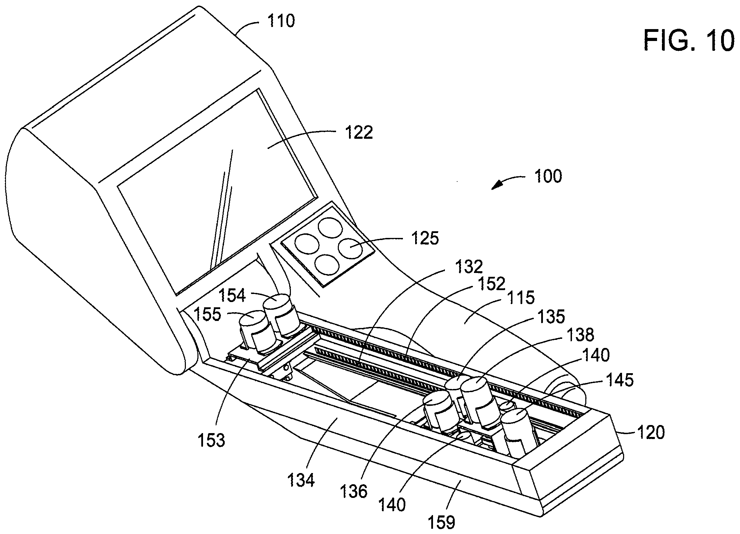

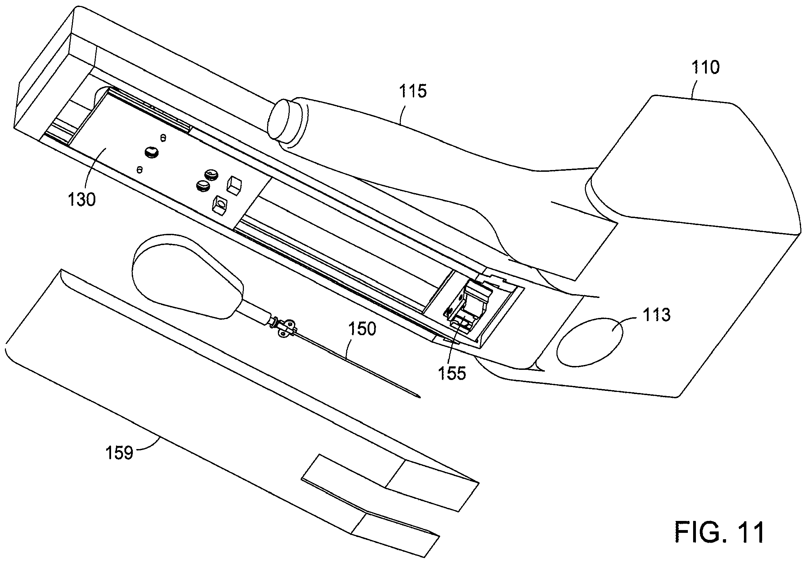

Figures 9-11 show another embodiment of an image guide portable device 100 for placing a catheter or needle into a lumen of a blood vessel. The device 100 has a console 110, a handle 115, and an actuator 120 for deploying the needle and the catheter. Figure 10 shows a top view of the device 100 with the cover of the actuator 120 removed. The console 110 includes an imaging machine, a monitor 122, and a controller for controlling the actuator 120. The console 110 may include one or more control buttons 125 for operating the device 100. The control buttons 125 may be positioned on the console 110 such that each is within the reach of the operator's hand holding the handle 115. In one embodiment, the device 100 may have two control buttons; one for starting the entire operation and one for emergency stop. The device 100 may also have multiple buttons to control the each step of the operation. For example, one for operating the imaging machine, one for injecting the needle and the catheter, and one for ejecting the needle from the device 100. In another embodiment, the handle 115 may have a rectangular shape and the control buttons are positioned for manipulation by the thumb of the hand gripping the handle 115. In yet another embodiment, the control buttons may be located on the housing for activation by the user's left hand. In this respect, the user may control the x and y motion of the device with the left hand while supporting the procedure with the right hand. The use of both hands would provide consistent pressure to ensure high fidelity with regard to imaging during operation of the device. In another embodiment still, the handle 115 may serve as a receptacle for a battery to provide power to the device 100. The battery may be any suitable energy storage unit, including disposable or rechargeable batteries. Figure 11 is a bottom view of the device 100, which is shown along with the catheter cartridge 150 and its protective casing 159.

The imaging machine may be an ultrasound machine or any suitable imaging machine for detecting a target such as the lumen of the vessel as described above. The ultrasound machine may send and receive signals to and from the central vein through the probe 113 located at the lower portion of the console 110. The ultrasound machine may analyze the ultrasound data and display the result on the monitor. Viewing the monitor, the operator may move the device 100 until desired position for the needle is in the target zone on the monitor. In another embodiment, the target zone in the vein may be determined by the programmable logic in the controller.

The controller is adapted to process the ultrasound data and control the actuator 120 to deploy the needle to the central vein. For example, the controller may include programmable logic to determine the distance to the lumen and to adjust the azimuth angle of the actuator 120 for penetration into the skin. The controller may also include programmable logic to cause the actuator 120 to deploy and/or retrieve the needle, guide wire, and the catheter.

The actuator 120 may be pivotally connected to the console 110. The actuator 120 may be pivoted relative to the console 110 to adjust the azimuth of the needle for insertion. The actuator 120 contains the mechanisms for deploying the needle, guide wire, and the catheter. Referring to Figures 10 and 11, the actuator 120 includes a carrier 130 for connection to the catheter cartridge 150, which includes the needle, guide wire, and the catheter. The carrier 130 is movable along a lower track 132 located on an inner portion of the actuator frame 134. The carrier 130 may include an injector motor 135 for moving the carrier 130 relative to the track 132. The carrier 130 may further include a guide wire motor 136 for activating the rollers in the cartridge 150 for inserting and retrieving the guide wire. Each of the motors may be operated in both the forward and the reverse directions. The carrier 130 may be equipped with a mechanism 138 for creating a vacuum in the needle to aspirate some blood into the needle. The carrier 130 may further include a cartridge engagement indicator 140 for engaging the cartridge 150. The indicator 140 activates a locking mechanism 145 when engaged with the cartridge. The locking mechanism 145 is adapted to connect and release the cartridge 150. The actuator 120 may also include a catheter conveyor 153 movable on an upper track 152 in the actuator frame 134. The catheter conveyor 153 has a motor 154 for moving the conveyor and a catheter gripper 155 for engaging and moving the catheter relative to the needle.

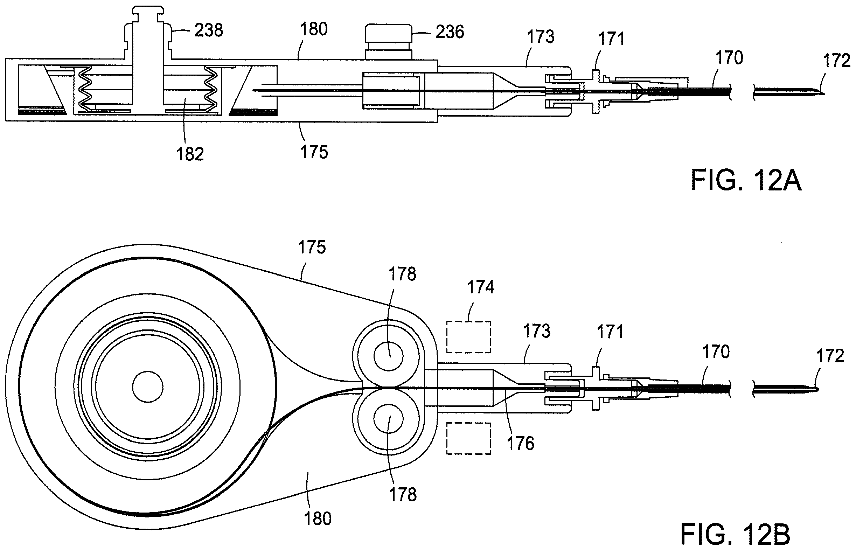

Figures 12A-B are side view and top view of the catheter cartridge 150, respectively. The cartridge 150 may be encased in a protective casing 159 and is movable relative to the protective casing 159. The protective casing 159 may be releasably connected to the frame 134 or other nonmoving portions of the actuator 120, and the catheter cartridge 150 is releasably connected to the carrier 130.

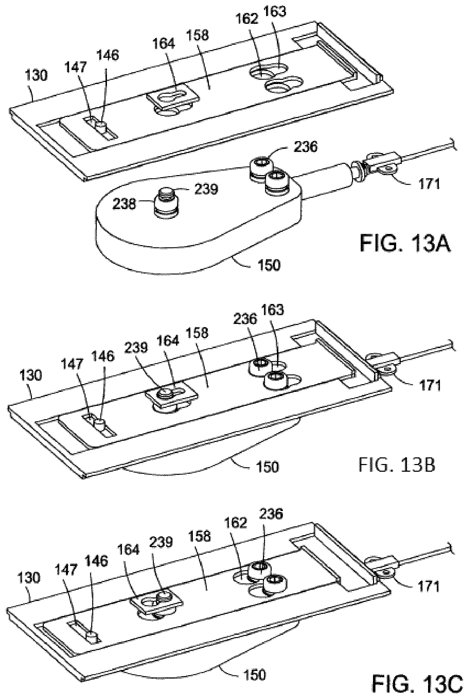

In one embodiment, the catheter cartridge 150 is connected to the carrier 130 via the connection 236 to the guide wire motor 136 and via the connection 238 to the vacuum mechanism 138. Referring to Figures 13A-C, the upper end of the vacuum mechanism connection 238 may include a neck 239 for engaging a locking plate 158 activatable by the locking mechanism 145 of the carrier 130. In one embodiment, the locking mechanism 145 has a pin 146 that is movable in a slot 147 of the locking plate 158. The locking plate 158 has an opening 162 to allow entry of the neck 236. Figure 13B shows the connections 236, 238 inserted into their respective openings 162. Each opening 162 has a recess 163 that is engageable with a smaller diameter section of the neck 236. To lock the catheter cartridge 150 in place, the locking plate 158 is shifted to move the recess 163 into engagement with smaller diameter neck section. The locking plate 158 may be shifted by moving the pin 146 in an arcuate path. In this embodiment, the pin 146 is rotated 90 degrees. Figure 13C shows the cartridge 150 locked into positioned and therefore, movable with the carrier 130.

Referring back to Figures 12A-B, the catheter cartridge 150 includes the catheter 170, the needle 172, and the housing 175 containing the guide wire 176. The catheter 170 is coaxially disposed around the shaft of the needle 172. The back portion of catheter includes extensions 171 such as a wing or shoulder for mating with the catheter gripper 155 of the catheter conveyor 153. The catheter 170 may be made of an elastomer or any suitable catheter material known to a person of ordinary skill. The hub 173 of the needle 172 may optionally include one or more sensors 174 for analyzing the bodily fluid in the hub 173. The sensors 174 may determine whether the fluid is arterial or venous blood. The housing 175 is connected to the hub 173 and includes the guide wire 176 and a guide wire feeder 178 such as a roller. As shown, two rollers 178 are provided, and at least one of the rollers 178 may be active. The active roller 178 is connectable to the drive shaft of the guide wire motor 136. The guide wire 176 may be wound around a pinion in the housing 175. In one embodiment, the tip of the guide wire 176 is pre-positioned in the bore of the shaft of the needle 172 before deployment. An exemplary guide wire 176 is a flexible "J" tipped guide wire. The interior of the housing 175 may function as a chamber 180 for collecting bodily fluid. In one embodiment, the housing 175 may include a diaphragm 182 which may be manipulated by the vacuum mechanism 138 to create a negative pressure in the chamber 180. For example, the diaphragm 182 may be manipulated to increase the volume in the chamber 180, thereby creating a vacuum effect in the chamber 180. The amount of the negative pressure created, *i.e.,* change in volume, may be controlled by the controller. In this respect, the negative pressure may be created in a step-wise manner or gradually over time.

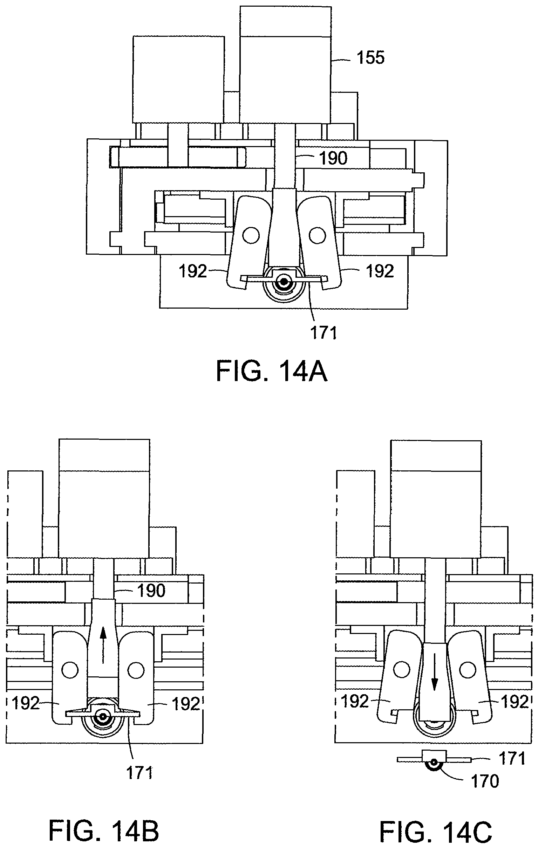

Figures 14A-C shows an embodiment of the catheter gripper 155. The catheter gripper 155 includes a piston 190 for activating the gripping elements 192. The piston 190 has a cone shaped portion and is axially movable. The gripping elements 192 are adapted to grip the extensions 171 of the catheter 170 and pivotable between an engaged position and a released position. Figure 14A shows the gripper elements 192 in the released position, wherein the piston 190 is partially extended such that the upper portion of the gripping elements 192 is pivoted inwardly to engage the cone shaped portion. To grip the extensions 171, the piston 190 is retracted such that the wider portion of the piston 192 pivots the upper portion of the gripping elements 192 outwardly, which in turn, pivots lower portion into engagement with the extensions 171 of the catheter 170. Figure 14B shows the gripping elements 192 in the engaged position. To release the catheter 170, the piston is fully extended to open the gripping elements 192 and urge the catheter 170 away from the device 100, as illustrated in Figure 14C.

An exemplary procedure for placing the catheter 170 using the device 100 will now be described. It should be noted that prior to performing the procedure, the desired area of operation on the patient should be prepped in the usual sterile fashion. A sterile field will be established using drapes and a sterile ultrasound media will be applied the general area to be determined by the operator. A sterile ultrasound cap made of thin plastic may be wrapped around the device. The cartridge prevents exposure to the sharp implements of the device.

In operation, a cartridge 150 is releasably connected to the actuator 120 of the device 100. The cartridge 150 engages the cartridge indicators 140, which in turn, activates the locking mechanism 145, whereby the locking plate 158 moves into engagement with the neck of the vacuum machine connection 238 and the guide wire connections 236. The device 100 is then positioned adjacent the patient. Using ultrasound imaging, the device 100 identifies the central vein 7 in the patient. From that image, a center point or target and an azimuth for the needle's descent will be determined by the controller. The target sets the floor or maximum penetration depth. Prior to insertion, the surgically prepped skin surface 8 will be exposed to an anesthetic. The anesthetic may be delivered manually or by an optional component of the device 100.

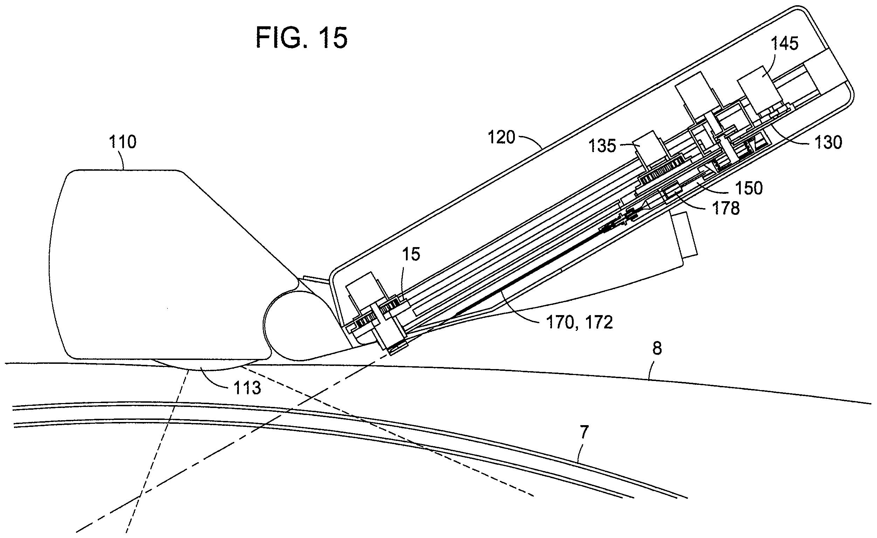

Figure 15 shows the device 100 with the actuator 120 pivoted to the proper angle for insertion of the needle 172 and the co-axially disposed catheter 170. From this position, the controller activates the actuator 120 to insert the needle 172. In this embodiment, the injector motor 135 is activated to inject the needle 172 by advancing the carrier 130 and the attached catheter cartridge 150 toward target. In this respect, the catheter 170 and the needle 172 are inserted into the vein 7 simultaneously.

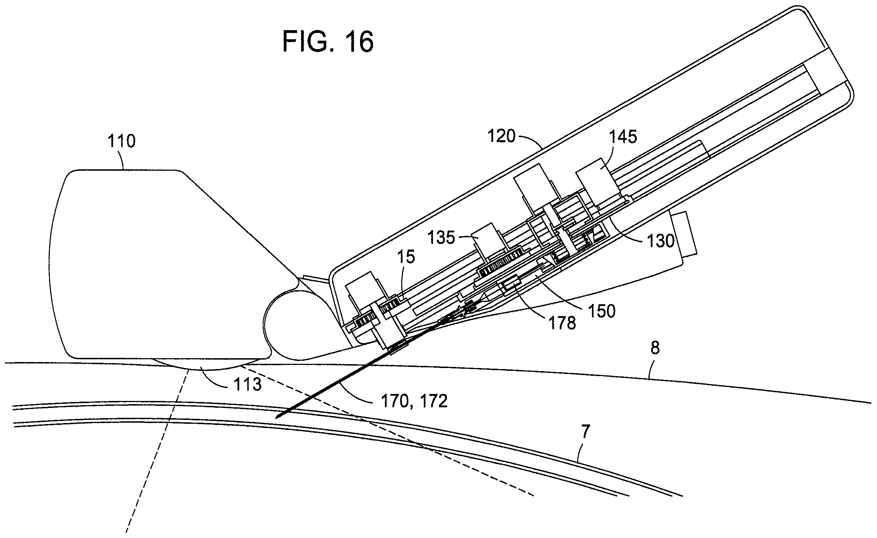

Figure 16 shows the tip of the needle 172 inside the vein 7. With the needle tip in place, the controller activates the vacuum mechanism 138 to expand the diaphragm 182 to aspirate a sample of bodily fluid into the needle 172. Aspiration may also remove air from the bore of the needle 172. The fluid sample may be analyzed by the sensor 174 in the needle hub 173 to confirm the fluid is venous fluid, e.g., venous blood. In another embodiment, fluid confirmation may be visually performed by the operator. In yet another embodiment, the device 100 may be equipped with a pressure sensor to detect the puncture of a blood vessel by the needle.

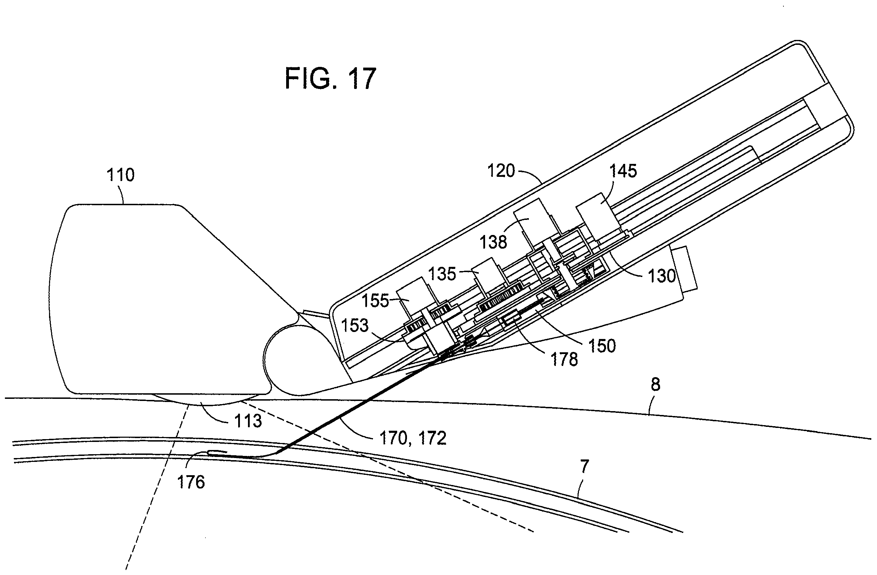

After insertion of the needle 172 and the catheter 170, the catheter conveyor 153 is retracted toward the carrier 130 and positioned over the catheter 170, as shown in Figure 17. The catheter gripper 155 is then activated to grip the extensions 171 on the catheter 170. Simultaneously, the guide wire motor 136 is activated to rotate the rollers 178 to advance the flexible "J" tipped guide wire 176 through the needle 172 and into the vein 7. It must be noted that these two steps may also be performed individually.

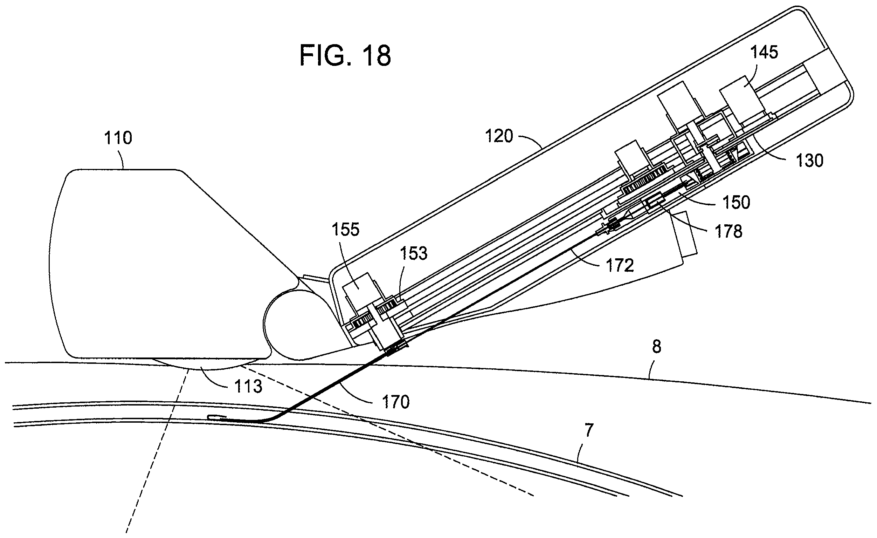

Referring to Figure 18, after the guide wire 176 has extended past the needle tip, the needle 172 is retracted and the catheter 170 is advanced over the guide wire 176. The needle 172 is retracted by retracting the carrier 130, and the catheter 170 is advanced by advancing the catheter conveyor 153. In this respect, the carrier 130 and the catheter conveyor 153 move in opposite directions, thereby separating the catheter 170 from the needle 172. The movements of the carrier 130 and the catheter conveyor 153 may be simultaneous or performed separately. The guide wire 176 is extended sufficiently as to ensure that the tip of the catheter 170 does not extend past the flexible "J" tip of the guide wire 176. In one embodiment, during needle retraction, the negative pressure aspiration is repeated to aspirate from within the catheter 170 as the needle 172 sheds the catheter 170. In this respect, air in the catheter 170 may be removed from the catheter 172. Retraction of the needle 172 moves the needle 172 back into the cartridge 150. After placement of the catheter 170, the guide wire 176 is retracted back into the housing 175 using the same roller system. In another embodiment, the catheter 170 may be advanced along the guide wire 176 before the needle 172 is retracted. Because the guide wire 176 may be retracted when the needle 172 is retracted, advancing the catheter 170 separately ensures the catheter 170 is properly positioned before needle retraction.

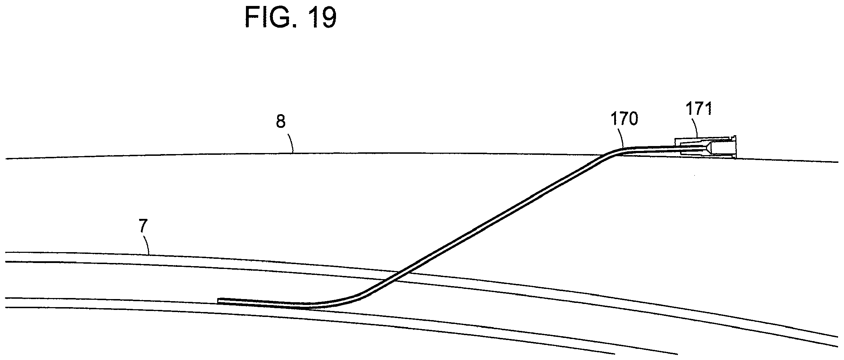

The operator may now activate the eject button to release the catheter gripper 155 and release the cartridge locking mechanism 145 on the carrier 130. In one embodiment, the eject button extends the piston 190 of the catheter gripper 155 to urge the cartridge 150 away from the actuator 120. The cartridge 150 may be ejected into an appropriate disposal unit. The device 100 may be used again with a new cartridge. Figure 19 shows one end of the catheter placed in the vein 7 and the other end on the skin surface 8.

It is contemplated that features of the described with respect to the embodiments of the present invention may be interchangeable between the embodiments. For example, the handle described in the embodiment of Figure 9 may be adapted for use with the embodiment described in Figure1. Additional features that may be interchangeable include, but not limited to, the features of the cartridges, including the vacuum mechanism, the gripper mechanism, imaging machine, drive motors, track systems. Other features may be suitably interchanged as is known to a person of ordinary skill in the art.

Although the embodiments of the robotic device are described for use in placing a central venous catheter, it must be noted that the robotic devices may be used in a variety of procedures. For example, the robotic devices may be used to introduce a medical device to perform any diagnostic or therapeutic procedure. The imaging capabilities of the robotic devices allow the introduction of a medical device to an intended target without direct vision of that target. Exemplary imaging capabilities may be selected from at least one of ultrasound, magnetic resonance imaging, computed tomography, and any suitable imaging mechanisms known to a person of ordinary skill in the art. The imaging capabilities may allow the robotic device to detect or delineate the geometry and position of the target anatomical structure. In this respect, an operator may rely on the imaging capability to position and aim the device for a specific location in the targeted structure and thereafter instruct the controller to actuate the actuator to introduce the medical device into the targeted structure. Additionally, the imaging capabilities may allow the operator to select a path for the needle to prevent the needle from accidentally puncturing adjacent structures.

In one embodiment, medical device may be a flexible or rigid tubular such as a needle, a cannula, or a catheter. In another embodiment, the medical device may be a solid elongated flexible or rigid member such as a guide wire. In yet another embodiment, the robotic device may be configured to simultaneously or sequentially introduce one or more medical devices into the targeted structure. For example, the robotic device may simultaneously inject a needle and a coaxially position catheter into a target vessel. In one embodiment, the medical device may be retrieved or at least temporarily remain in place after completion of the procedure.

In one embodiment, the robotic device may be used to introduce a medical device to the target structure to facilitate sampling, monitoring, patency supporting, infusing, draining, and/or delivering another medical device or chemical compound to the target structure.

In one embodiment, the robotic device may be used to introduce a medical device to an artery or vein such as central or peripheral vein or artery.

In another embodiment, the robotic device may be used to introduce a medical device to the epidural and or the Intrathecal space through an intervertebral disc in the spinal column.

In another embodiment, the robotic device may be used to introduce a medical device into the mammary tissue; pleural space; pericardial space; thyroid or parathyroid capsule and or tissue; thymus capsule and or tissue; hepatic capsule or tissue; choleangial tissue or duct; and pancreatic capsule, tissue, or duct. Additional suitable anatomical structure include spleen capsule or tissue; renal capsule or tissue; ureteral tissue or duct; ovarian capsule or tissue; fallopian tissue or duct; testicular capsule or tissue; prostatic capsule or tissue; vaginal or cervical tissue; urinary bladder capsule or tissue; bone marrow tissue; spine containing nerve tissue; spinal osteo tissue; and meninges.

In another embodiment, the robotic device may be used to introduce a medical device into the stomach, duodenum, jejunum, ileum, and the ascending, transverse, or descending or sigmoid colon.

In another embodiment, the robotic device may be used to introduce a medical device into the intrauterine space.

In another embodiment, the robotic device may be used to introduce a medical device into the intra abdominal space.

In another embodiment, the robotic device may be used to introduce a medical device to secure an airway into the intratracheal space.

While the foregoing is directed to embodiments of the present invention, other and further embodiments of the invention may be devised without departing from the basic scope thereof, and the scope thereof is determined by the claims that follow.

## Claims

1. An image guided device for introducing a medical device comprising a needle (60), a catheter (55) and a guide wire (70), into a body structure, comprising:
an imaging device for acquiring information of a location of the body structure;
an actuator (20; 120) for deploying the medical device, wherein the actuator is pivotally connected to a console (10) by a support arm (17), and wherein the actuator comprises:
a first injector motor (31) for advancing or retracing the needle; and
a second injector motor (32) for advancing or retracing the catheter relative to the needle;
a catheter cartridge (50; 150) releasably attached to the actuator, the catheter cartridge containing the medical device, which includes the guide wire (70), wherein the guide wire is partially disposed in the needle (60), wherein the catheter (55) is coaxially disposed around a shaft of the needle, and wherein the guide wire (70) is wound around a spool (65) of the cartridge and adapted to be spooled out along with an insertion of the needle (60);
and a controller for receiving and processing information of the location of the body structure and in response controlling the first injector motor to advance or retract the needle and the second injector motor to advance or retract the catheter relative to the needle to place the medical device into the body structure.

2. The apparatus of claim 1, wherein the apparatus is portable.

3. The apparatus of claims 1 or 2, wherein the first injector motor is a linear motor.

4. The apparatus of claim 3, wherein the second injector motor is a linear motor.

5. The apparatus of claim 4, further comprising a rotary motor (33) adapted to advance or retract the guide wire (70) through the needle (60).

6. The apparatus of any of claims 1-5, wherein the catheter is configured for simultaneous insertion with the needle.

7. The apparatus of any of claims 1-6, further comprising an aspiration device.

8. The apparatus of claim 7, wherein the aspiration device comprises a vacuum mechanism.

9. The apparatus of any of claims 1-8, wherein the console (110) is coupled to the imaging device and a handle (115).

10. The apparatus of claim 9, wherein the actuator is pivotable relative to the console to position the catheter.

11. The apparatus of any of claims 1-10, wherein the imaging device is capable of projecting an image of the body structure on a monitor.

## Patentansprüche

1. Bildgeführte Vorrichtung zum Einführen eines medizinischen Geräts, das eine Nadel (60), einen Katheter (55) und einen Führungsdraht (70) umfasst, in eine Körperstruktur, umfassend:
eine Bildgebungsvorrichtung zum Erfassen von Informationen eines Ortes der Körperstruktur;
einen Aktuator (20; 120) zum Anwenden des medizinischen Geräts, wobei der Aktuator durch einen Stützarm (17) schwenkbar mit einer Konsole (10) verbunden ist und wobei der Aktuator umfasst:
einen ersten Injektormotor (31) zum Vorwärtsbewegen oder Zurückziehen der Nadel; und
einen zweiten Injektormotor (32) zum Vorwärtsbewegen oder Zurückziehen des Katheters relativ zur Nadel;
ein Kathetermodul (50; 150), das lösbar am Aktuator befestigt ist, wobei das Kathetermodul das medizinische Gerät enthält, welches den Führungsdraht (70) aufweist, wobei der Führungsdraht teilweise in der Nadel (60) angeordnet ist, wobei der Katheter (55) koaxial um eine Achse der Nadel herum angeordnet ist und wobei der Führungsdraht (70) um eine Spule (65) des Moduls gewickelt ist und dafür ausgelegt ist, zugleich mit einem Einsetzen der Nadel (60) abgewickelt zu werden;
und eine Steuereinrichtung zum Empfangen und Verarbeiten von Informationen des Ortes der Körperstruktur und Steuern, in Reaktion darauf, des ersten Injektormotors, um die Nadel vorwärtszubewegen oder zurückzuziehen, und des zweiten Injektormotors, um den Katheter relativ zur Nadel vorwärtszubewegen oder zurückzuziehen, um das medizinische Gerät in der Körperstruktur anzuordnen.

2. Vorrichtung nach Anspruch 1, wobei die Vorrichtung tragbar ist.

3. Vorrichtung nach Anspruch 1 oder 2, wobei der erste Injektormotor ein Linearmotor ist.

4. Vorrichtung nach Anspruch 3, wobei der zweite Injektormotor ein Linearmotor ist.

5. Vorrichtung nach Anspruch 4, welche ferner einen Rotationsmotor (33) umfasst, der dafür ausgelegt ist, den Führungsdraht (70) durch die Nadel (60) vorwärtszubewegen oder zurückzuziehen.

6. Vorrichtung nach einem der Ansprüche 1-5, wobei der Katheter für ein gleichzeitiges Einsetzen mit der Nadel ausgebildet ist.

7. Vorrichtung nach einem der Ansprüche 1-6, welche ferner eine Absaugvorrichtung umfasst.

8. Vorrichtung nach Anspruch 7, wobei die Absaugvorrichtung einen Vakuummechanismus umfasst.

9. Vorrichtung nach einem der Ansprüche 1-8, wobei die Konsole (110) mit der Bildgebungsvorrichtung und einem Griff (115) gekoppelt ist.

10. Vorrichtung nach Anspruch 9, wobei der Aktuator bezüglich der Konsole schwenkbar ist, um den Katheter zu positionieren.

11. Vorrichtung nach einem der Ansprüche 1-10, wobei die Bildgebungsvorrichtung in der Lage ist, ein Bild der Körperstruktur auf einen Monitor zu projizieren.

## Revendications

1. Dispositif guidé par images pour l'introduction d'un dispositif médical, comprenant une aiguille (60), un cathéter (55) et un fil de guidage (70), dans une structure corporelle, comprenant :
un dispositif d'imagerie pour acquérir des informations sur un emplacement de la structure corporelle ;
un actionneur (20 ; 120) pour déployer le dispositif médical, l'actionneur étant connecté de manière pivotable a une console (10) par un bras support (17) et l'actionneur comprenant :
un premier moteur à injecteur (31) pour avancer ou rétracter l'aiguille ; et
un second moteur à injecteur (32) pour avancer ou rétracter le cathéter par rapport à l'aiguille ;
une cartouche de cathéter (50 ; 150) fixée de manière dissociable à l'actionneur, la cartouche de cathéter contenant le dispositif médical qui inclut le fil de guidage (70), le fil de guidage étant partiellement disposé dans l'aiguille (60), le cathéter (55) étant disposé coaxialement autour d'un axe de l'aiguille et le fil de guidage (70) étant enroulé autour d'une bobine (65) de la cartouche et apte à être déroulé lors d'une insertion de l'aiguille (60) ;
et un contrôleur pour recevoir et traiter des informations sur l'emplacement de la structure corporelle et commander en réaction au premier moteur à injecteur d'avancer ou de rétracter l'aiguille et au second moteur à injecteur d'avancer ou de rétracter le cathéter par rapport à l'aiguille pour placer le dispositif médical dans la structure corporelle.

2. Appareil selon la revendication 1, cet appareil étant portable.

3. Appareil selon la revendication 1 ou 2, dans lequel le premier moteur à injecteur est un moteur linéaire.

4. Appareil selon la revendication 3, dans lequel le second moteur à injecteur est un moteur linéaire.

5. Appareil selon la revendication 4, comprenant en outre un moteur rotatif (33) apte à avancer ou à rétracter le fil de guidage (70) à travers l'aiguille (60).

6. Appareil selon l'une quelconque des revendications 1 à 5, dans lequel le cathéter est conçu pour être inséré en même temps que l'aiguille.

7. Appareil selon l'une quelconque des revendications 1 à 6, comprenant en outre un dispositif d'aspiration.

8. Appareil selon la revendication 7, dans lequel le dispositif d'aspiration comprend un mécanisme de vide.

9. Appareil selon l'une quelconque des revendications 1 à 8, dans lequel la console (110) est couplée au dispositif d'imagerie et à une poignée (115).

10. Appareil selon la revendication 9, dans lequel l'actionneur est pivotable par rapport à la console pour positionner le cathéter.

11. Appareil selon l'une quelconque des revendications 1 à 10, dans lequel le dispositif d'imagerie est capable de projeter une image de la structure corporelle sur un moniteur.
